# EUROPEAN PATENT APPLICATION

(11) **EP 4 534 078 A1**
(43) Date of publication of application: **09.04.2025**
(21) Application number: 23816334.9
(22) Date of filing: 30.05.2023
(51) Int. Cl.: A61K 9/51, A61K 47/69, A61K 38/00, A61P 31/04, C12N 15/115

(54) **ANTIMICROBIAL PROTEIN CARRIER BASED ON GOLD NANOPARTICLE-APTAMER CONJUGATE AND FABRICATION METHOD THEREFOR**

(30) Priority: 30.05.2022 KR 20220066226; 26.12.2022 KR 20220184356
(71) Applicant: NES BIOTECHNOLOGY CO., LTD., Seoul 06974 (KR)
(72) Inventor: LEE, Kangseok, Gwangju-si, Gyeonggi-do 12765 (KR); BAE, Jeehyeon, Gwangju-si, Gyeonggi-do 12765 (KR); YEOM, Jihyun, Yangju-si, Gyeonggi-do 11426 (KR); SIM, Sehoon, Suwon-si, Gyeonggi-do 16493 (KR); JOO, Minju, Seoul 03638 (KR); BAE, Dayeong, Pyeongtaek-si, Gyeonggi-do 17943 (KR); LEE, Jaemin, Seoul 01721 (KR)
(74) Representative: Dehns
(86) International application number: PCT/KR2023/007375
(87) International publication number: WO 2023/234673

(57) **Abstract**

The present invention relates to an antimicrobial peptide delivery vehicle capable of efficiently delivering an antimicrobial peptide into cells and inducing its expression. The delivery vehicle comprises a gold nanoparticle and an aptamer that specifically binds to the antimicrobial peptide. The invention also encompasses a method for preparing the delivery vehicle and an antimicrobial composition comprising the same.

## Description

### [Technical Field]

The present invention relates to an antimicrobial peptide delivery vehicle based on a gold nanoparticle-aptamer conjugate and a method for producing the same, and more specifically, to an antimicrobial peptide delivery vehicle having improved delivery capability of an antimicrobial peptide into cells by preparing and conjugating an aptamer specific for an antimicrobial peptide to a gold nanoparticle and a method for producing the same.

### [Background Art]

Bacterial resistance to existing antimicrobial agents is increasing every year and is one of the major problems in the global health field. Most of the current research on infectious diseases worldwide is focused on the development of antibiotics that selectively inhibit and control the metabolic or biosynthetic pathways of pathogens and the development of vaccines by culturing attenuated pathogens. However, existing antibiotics often show side effects and toxicity by simultaneously inhibiting enzymes that control the metabolic and biosynthetic pathways of the host, and their use is limited due to resistance issues. Therefore, the development of a new paradigm that can effectively control infectious diseases in the early stages is urgent, and antimicrobial peptides (AMPs) are receiving attention as an example.

Most AMPs are cationic peptides with an amphipathic structure that selectively target bacterial membranes through electrostatic forces. Among them, WLBU2 was rationally designed by amino acid substitution of the precursor peptide to have an ideal amphipathic helical structure to maximize antibacterial properties while minimizing epithelial cell toxicity. However, for practical use, the delivery of these antimicrobial peptides has the disadvantages of causing toxicity that can harm normal cells, the inability to store the delivered drugs for a long time, and low delivery accuracy.

**In** a previous study, the inventors of the present invention manufactured a nanoparticle complex for antibody delivery, and the antibody delivery vehicle based on the gold nanoparticle-aptamer complex was confirmed to have improved intracellular antibody delivery ability and excellent stability. Accordingly, there is a demand for the development of an excellent nanoparticle-based delivery vehicle that has excellent drug delivery ability and improved problems such as toxicity and storage stability that antimicrobial peptides may have.

### [Disclosure]

### [Technical Problem]

Therefore, the inventors of the present invention have made extensive research efforts to develop a gene delivery technology that can efficiently deliver and induce expression of an antimicrobial peptide (WLBU2) into cells without toxicity. As a result, by utilizing the SELEX technique, an aptamer with significantly higher binding affinity to the antimicrobial peptide was developed, and in order to effectively deliver the aptamer into a living body, a complex was created by covalently bonding it with nanoparticles (AuNP), and then combined with the antimicrobial peptide (WLBU2), thereby completing the present invention that can stably deliver the antimicrobial peptide into the body.

Therefore, the present invention is directed to the development of a target aptamer for an antimicrobial peptide, which can bind to nanoparticles, and to the provision of an antimicrobial peptide delivery vehicle comprising a gold nanoparticle material.

The present invention also aims to provide a method for producing an antimicrobial peptide delivery vehicle, comprising a step of producing an aptamer specific to an antimicrobial peptide and a step of binding the aptamer to a gold nanoparticle.

The present invention also aims to provide an antimicrobial composition comprising the antimicrobial peptide delivery vehicle.

### [Technical Solution]

To address the above-described objects, the present invention provides an antimicrobial peptide delivery vehicle comprising a target aptamer for WLBU2, an antimicrobial peptide capable of binding to nanoparticles, produced through the SELEX technique, and a gold nanoparticle material.

The present invention also provides a method for producing an antimicrobial peptide delivery vehicle, including a step of producing an aptamer specific to the antimicrobial peptide WLBU2 and a step of binding the aptamer to gold nanoparticles.

The present invention also provides an antimicrobial composition comprising the antimicrobial peptide delivery vehicle.

Hereinafter, the present invention will be described in detail.

As one aspect of the present invention, the present invention relates to an antimicrobial peptide delivery vehicle comprising a target aptamer for an antimicrobial peptide capable of binding to nanoparticles and a gold nanoparticle material.

In the present invention, the term "nanoparticle (NP, nanoparticle)" refers to a metal particle with a diameter in the nanometer range, preferably a "gold nanoparticle." The gold nanoparticle in the present invention facilitates penetration into target cells and enables the delivery of peptides into cells.

The gold nanoparticles are not only easy to manufacture in the form of stable particles, but can also be varied in size from 0.8 nm to 200 nm to suit various purposes. **In** addition, gold can modify its structure by combining with various types of molecules, such as peptides, proteins, and nucleic acids, and reflects light at various wavelengths, which can be used to easily identify its location within a cell. **In** addition, gold nanoparticles are harmless to the human body and have high biocompatibility, unlike heavy metals such as manganese, aluminum, cadmium, lead, mercury, cobalt, nickel, and beryllium.

When the diameter of gold nanoparticles exceeds 100 nm, their nanoparticle characteristics are lost, the bond between the gold surface, which lacks nanomaterial properties, and functional groups such as thiol groups becomes weak, and gold nanoparticles with diameters outside the range of 10-20 nm cause cytotoxicity. Therefore, it is preferable that the gold nanoparticles of the present invention have a diameter of 10-20 nm.

**In** the present invention, the term "aptamer" refers to a short-length oligomer, a single-stranded nucleic acid that forms a stable structure and exhibits the characteristic of binding to a target substance with high binding affinity and specificity. It forms a characteristic three-dimensional structure through intramolecular hydrogen bonds, enabling specific binding to the target via this spatial structure. Aptamers can be developed using SELEX (Systematic Evolution of Ligands by Exponential Enrichment), and due to their high affinity and specificity for a target, it is possible to develop aptamers capable of binding to various targets, including proteins, sugars, nucleic acids, and metal ions.

The antibacterial protein WLBU2 of the present invention can have at least 70%, at least 80%, at least 90%, at least 95%, at least 97%, or at least 99% sequence identity with the amino acid sequences from SEQ ID NO: 2 to SEQ ID NO: 5 or from SEQ ID NO: 1 to SEQ ID NO: 4. The known WLBU2 protein structure is mainly composed of 13 Arg residues and 8 Val residues, and exhibits amphipathic properties, including a structure where three Trp residues located on the hydrophobic side are separated by at least 7 amino acids.

The WLBU2 of the present invention may have antibacterial activity against Gram-negative bacteria, Gram-positive bacteria, antibiotic-resistant Gram-negative bacteria, and antibiotic-resistant Gram-positive bacteria.

The above "gram-negative bacteria" include all types of gram-negative bacteria containing endotoxin, including, but not limited to, strains from the genus *Escherichia,* the genus *Pseudomonas,* the genus *Acinetobacter,* the genus *Salmonella,* the genus *Klebsiella,* the genus *Neisseria,* the genus *Enterobacter,* the genus *Shigella,* the genus *Moraxella,* the genus *Helicobacter,* the genus *Stenotrophomonas,* the genus *Bdellovibrio,* and the genus *Legionella.*

The above 'Gram-positive bacteria' may include, but are not limited to, strains of the genus Staphylococcus, Enterococcus, Streptococcus, and Clostridium.

In addition, "antibiotic-resistant gram-positive bacteria" may include, for example, methicillin-resistant gram-positive bacteria, carbapenem-resistant gram-positive bacteria, vancomycin-resistant gram-positive bacteria, macrolide-resistant gram-positive bacteria, and multidrug-resistant gram-positive bacteria. The "antibiotic-resistant gram-negative bacteria" may include, for example, streptomycin-resistant gram-negative bacteria, colistin-resistant gram-negative bacteria, carbapenem-resistant gram-negative bacteria, chloramphenicol-resistant gram-negative bacteria, tetracycline-resistant gram-negative bacteria, cefotaxime-resistant gram-negative bacteria, extended spectrum beta-lactamase (ESBL)-producing gram-negative bacteria, tigecycline-resistant gram-negative bacteria, and multidrug-resistant gram-negative bacteria. These examples are not limiting.

More specifically, the bacteria against which the antimicrobial peptide of the present invention exhibits antimicrobial activity include *Pseudomonas aeruginosa, Acinetobacter baumannii, Streptococcus gordonii, Fusobacterium nucleatum, Porphyromonas gingivalis, Francisella tularensis, Yersinia pestis, Burkholderia pseudomallei, Staphylococcus aureus,* MRSA *(Methicillin-Resistant Staphylococcus aureus), Enterococcus faecium, Klebsiella pneumoniae, Enterobacter cloacae, Escherichia coli,* and one or more species of *Candida.*

The antimicrobial protein delivery vehicle of the present invention comprises a gold nanoparticle and an aptamer that binds to the antimicrobial peptide to deliver the antimicrobial peptide to a target cell. The aptamer included in the delivery vehicle of the present invention comprises a nucleic acid sequence that specifically binds to the antimicrobial peptide WLBU2. The aptamer is bound to the surface of the gold nanoparticle and mediates the binding between the antimicrobial protein and the gold nanoparticle.

The antimicrobial protein delivery vehicle of the present invention comprises gold nanoparticles to which an aptamer specific to the antimicrobial peptide WLBU2 is bound. This vehicle enhances the cell delivery efficiency of the antimicrobial peptide and can be utilized as an antimicrobial agent employing WLBU2.

As one aspect of the present invention, the present invention relates to a method for producing an antimicrobial protein delivery vehicle, comprising the steps of developing an aptamer specific to WLBU2 and combining the aptamer with gold nanoparticles.

As another aspect of the present invention, the present invention relates to an antimicrobial composition comprising the antimicrobial protein delivery vehicle.

As used herein, the term "antimicrobial composition" refers to a composition having an activity of inhibiting the growth of microorganisms such as bacteria or fungi, and may include all forms used in various fields requiring an antimicrobial effect, and may be in the form of, for example, a pharmaceutical, cosmetic, quasi-drug, veterinary medicine, food additive, or feed additive.

The antimicrobial composition comprises the antimicrobial protein delivery vehicle of the present invention and may be widely used as an antimicrobial agent, antiviral agent, and antifungal agent. Its scope of application includes viruses that are harmful to plants, animals, and humans. The antibacterial targets of the antimicrobial composition include gram-negative bacteria, gram-positive bacteria, antibiotic-resistant gram-negative bacteria, and antibiotic-resistant gram-positive bacteria, as well as molds, yeast, and protozoa. The antimicrobial composition of the present invention may be used alone or in combination with other antibiotics depending on the intended use. In addition to pharmaceutical applications, it can also be effectively used as a food additive, cosmetic, ointment, or injection.

### [Advantageous Effects]

The present invention is an antibacterial protein delivery vehicle comprising gold nanoparticles to which an aptamer specific to the antibacterial peptide WLBU2 is bound. This vehicle enhances the cell delivery ability of the antibacterial peptide and maximizes its antibacterial effect on the target, making it suitable for use as an antibacterial agent utilizing an antibacterial protein.

### [Description of Drawings]

FIG. 1 is a schematic diagram illustrating the process of developing a target aptamer for the antibacterial peptide of the present invention.

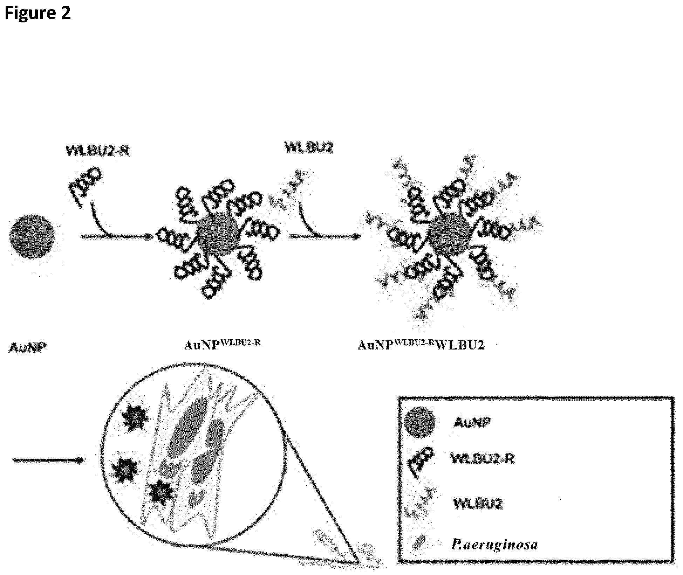

FIG. 2 is a schematic diagram illustrating the process of manufacturing an antimicrobial peptide delivery vehicle, AuNP^{WLBU2-R}, using the WLBU2-R aptamer that specifically binds to the antibacterial peptide of the present invention.
FIGs. 3 and 4 are diagrams showing the results of determining the number of aptamers (WLBU2-R) functionalized on gold nanoparticles and the number of peptides bound to them.
FIG. 5 is a diagram showing the results of confirming the survival rate based on the delivery efficiency and antimicrobial effect when delivering an antibacterial peptide using AuNP^{WLBU2-R} after bacterial infection in mice.

### [Best modes of the Invention]

Hereinafter, the present invention will be described in detail with reference to examples to help in understanding the present invention. However, examples according to the present invention may be modified in a variety of different forms, and it should not be construed that the scope of the present invention is limited to the following examples. The examples of the present invention are provided to more completely explain the present invention to those of ordinary skill in the art.

### Example 1. Minimum inhibitory concentration (MIC) test of strains for antimicrobial activity test

An experiment was conducted to determine the minimum inhibitory concentration (MIC) for multi-resistant strains in an antimicrobial activity test. *Pseudomonas aeruginosa, Acinetobacter baumannii,* and *Staphylococcus aureus* were cultured in LB medium at 1 × 10³ colony-forming units (CFU)/ml. Each strain was treated with WLBU2 peptide and antibiotics, such as ampicillin and erythromycin, at concentrations of 2, 4, 8, 16, 32, 64, 128, 256, and 512 µM, respectively, and then incubated at 37°C for 18 to 24 hours.

The minimum inhibitory concentration (MIC) was determined as the lowest O.D.600 value of each medium. As a result, it was confirmed that the antimicrobial peptide of the present invention exhibited the lowest MIC value for each strain compared to the antibiotics (Table 1).

**[Table 1]**

| | Ampicillin | Erythromycin | WLBU2 |
|---|---|---|---|
| Pseudomonas aerusinosa | >64µM | >64µM | 4µM |
| Acinetobacter baumanii | >256µM | >256µM | 8µM |
| Staphylococcus aureus | >256µM | >128µM | 4µM |

### Example 2. Development of WLBU2 aptamer using SELEX technique

### 2-1. Development of antimicrobial peptide target aptamer using SELEX technique

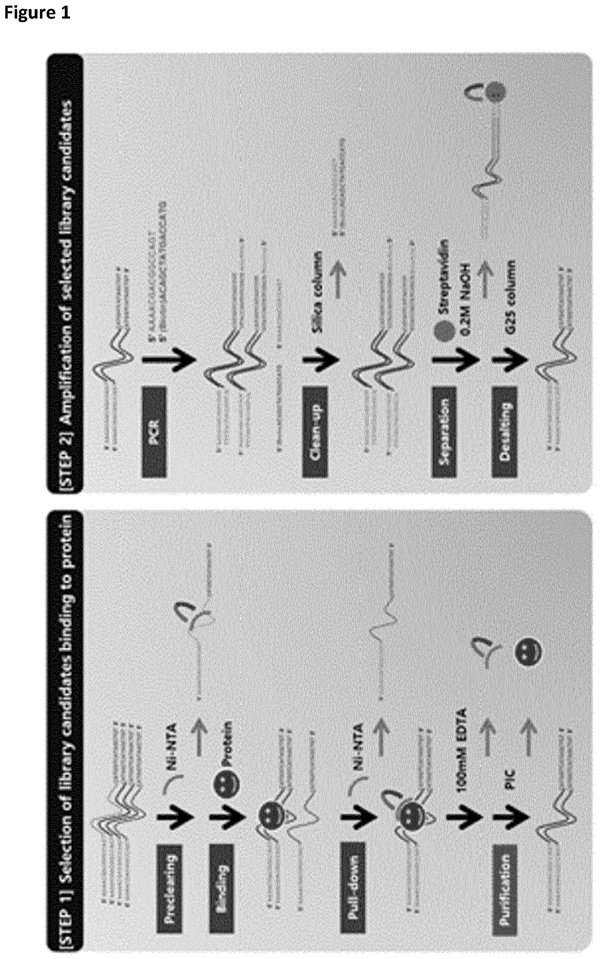

Aptamers specific to the antimicrobial peptide WLBU2 were developed using the SELEX (Systematic Evolution of Ligands by Exponential Enrichment) method. The SELEX process was repeated a total of 11 times, and the optimal aptamers with high binding affinity to WLBU2 were selected. The aptamer selection process was conducted according to the procedure illustrated in FIG. 1.

### 2-2. List of four representative target aptamers developed

Among all the sequences confirmed through sequencing, four representative sequences showing a fold change greater than 2-fold in the samples binding to WLBU2 compared to the control group were developed. The sequences and their fold changes are listed in Table 2 below.

**[Table 2]**

| SEQ No. | ID | Sequences | FoldChange |
|---|---|---|---|
| 1 | WLBU2-R | AAAACGACGGCCAGTGCGCGCGCTTTGCTTG TGTGAGCATTGCTTTTGCATTATTGGTTTGGT TGGTGTGGTTCGTGCGCGCCATGGTCATAGC CTGT | 2.2 |
| 2 | WLBU2-R-1 | AAAACGACGGCCAGTCTTTCGCGGGGTCTGG GGTTGGGGTTGTGGGTTAATTGCACTTCGAA ATGCATGGTCATAGCTGT | 3.28 |
| 3 | WLBU2-R-2 | AAAACGACGGCCAGTGGCTGGTAGGTTTGGG TGGTTTGGTTGGGATTCGGTTGGTTGGTACCA GCCATGGTCATAGCTGT | 3.67 |
| 4 | WLBU2-R-3 | AAAACGACGGCCAGTGCGCGCGCTTTGCTTG TGTGAGCATTGCTTTTGCATTATTGGTTTGGT TGGTGTGGTTCGTGCGCGCCATGGTCATAGC TGT | 2.7 |

### Example 3. Preparation of WLBU2-R Functionalized Gold Nanoparticles (AuNP^{WLBUZR})

An antimicrobial peptide delivery vehicle AuNP^{WLBU2-R} was prepared using the WLBU2-R aptamer that binds to the WLBU2 antimicrobial peptide.

### 3-1. WLBU2-R pretreatment

In order to prepare the antimicrobial peptide delivery agent according to the present invention, an aptamer was developed using the SELEX technique according to the method described in Example 2. Among the developed aptamers, WLBU2-R, an oligonucleotide modified with a thiol group at its 3'-terminal and consisting of the base sequence (SH) 5'-AAAACGACGGCCAGTCCATCCATTGGCTGGGTTAATCTTTATTGGCTGGGATT AACTGGATGGTGCATGGTCATAGCTGTAAAAAAAAAA-3' (SEQ ID NO: 1), was selected. The dried WLBU2-R was dissolved in water to a final concentration of 100 µM. Then, 20 µl of 3 M sodium acetate (pH 5.2) and 30 µl of 1 N DTT (dithiothreitol) were added to 150 µl of the oligonucleotide solution, and the mixture was reacted at room temperature for 60 minutes. To remove DTT containing unwanted thiol molecules, 200 µl of ethyl acetate was added, mixed, and the supernatant was removed by centrifugation. This process was repeated three times. Finally, oligo WLBU2-R was precipitated using the ethanol precipitation method.

### 3-2. Preparation of WLBU2-R Functionalization of Gold Nanoparticles (AuNP^{WLBU2-R})

The WLBU2-R precipitated through the process described in 3-1 was dissolved in water, added to gold nanoparticles, and combined using the salt-aging method. Specifically, WLBU2-R was added to 7 nM gold nanoparticles (AuNP:WLBU2-R = 1:300) and mixed thoroughly. NaCl was then added to a concentration of 0.1 M, and the mixture was incubated for 4 hours. After 4 hours, NaCl was added to a concentration of 0.2 M and mixed for an additional 4 hours. Following this, NaCl was added to a concentration of 0.3 M and mixed for 12 hours. After 12 hours, the WLBU2-R and gold nanoparticle mixture was centrifuged at ~10,000 × g for 20 minutes, and the pellet was collected. The unreacted aptamers in the supernatant were removed. This process was repeated three times. The final gold nanoparticle-WLBU2-R complex was dispersed in 10 mM sodium phosphate buffer (pH 7.4) containing 0.1 M NaCl.

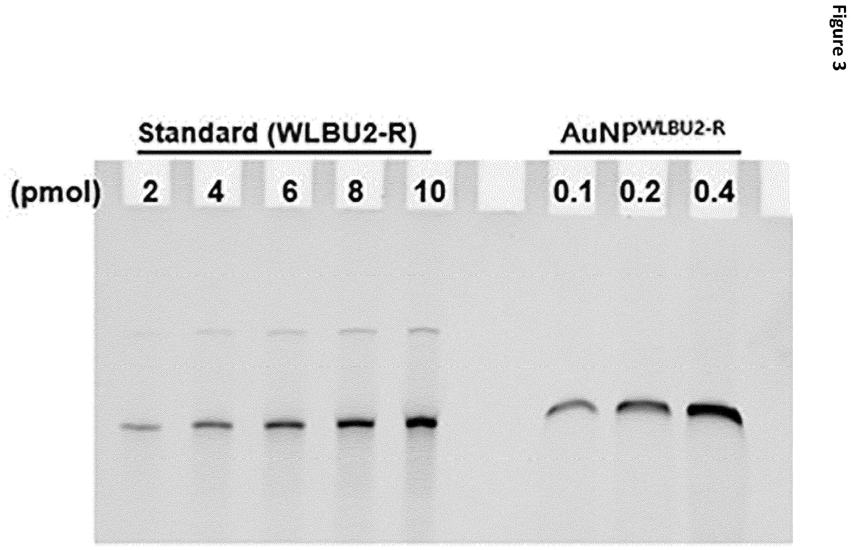

The prepared gold nanoparticle-WLBU2-R conjugate was analyzed by electrophoresis on a 10% acrylamide 8 M urea gel. It was confirmed that 45-50 WLBU2-R molecules were bound to a single gold nanoparticle (FIG. 3).

### 3-3. Preparation of AuNP^{WLBU2-R}-WLBU2 conjugate

AuNP^{WLBU2-R} and WLBU2 peptide conjugates were prepared as described in section 3-2. To prevent the formation of secondary structures in the functionalized gold nanoparticles, AuNP^{WLBU2-R} was pre-incubated at 80°C for 5 minutes. After cooling at room temperature for 5 minutes, PBS and WLBU2 peptide were added, followed by 0.01 M MgCl₂ to facilitate the binding of the peptide to the functionalized gold nanoparticles. The mixture was reacted at room temperature for 10 minutes.

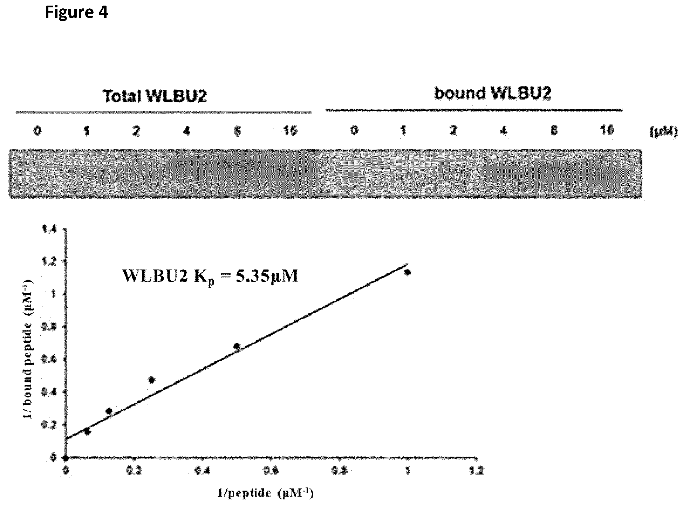

The resulting conjugate was centrifuged at 15,000 × g for 10 minutes, and the supernatant was removed. The binding was confirmed by electrophoresis using a Tricine-SDS-PAGE gel. The results showed that 5-6 peptides were bound to each gold nanoparticle (FIG. 4).

### Example 4. Confirmation of the delivery ability of WLBU2 antimicrobial peptide by AuNP^{WLBU2-R} in a small animal model

In animals infected with bacteria, experiments were conducted to compare the delivery ability of antimicrobial peptide transporters by injecting WLBU2 and AuNP^{WLBU2}-WLBU2, respectively. For the experiments, animals (mice) were housed in an animal room maintained at a humidity of 30-40% and a temperature of 22±1°C. The room followed a 12-hour light/dark cycle. The animal protocol was approved by the Support Center for Animal Experiments at Chung-Ang University, and the animals were handled in accordance with the approved protocol.

Six-week-old BALB/c mice (Central Lab Animal Inc, Korea) were injected with *Pseudomonas aeruginosa* PAO1 strain (1.5 × 10⁶ CFU), followed by intravenous (I.V.) injections of WLBU2 (4 mg/kg) and AuNP^{WLBU2-R}(10 nM)-WLBU2 (4 mg/kg) at 1 hour and 2.5 hours post-infection, respectively. The survival of the mice was subsequently observed.

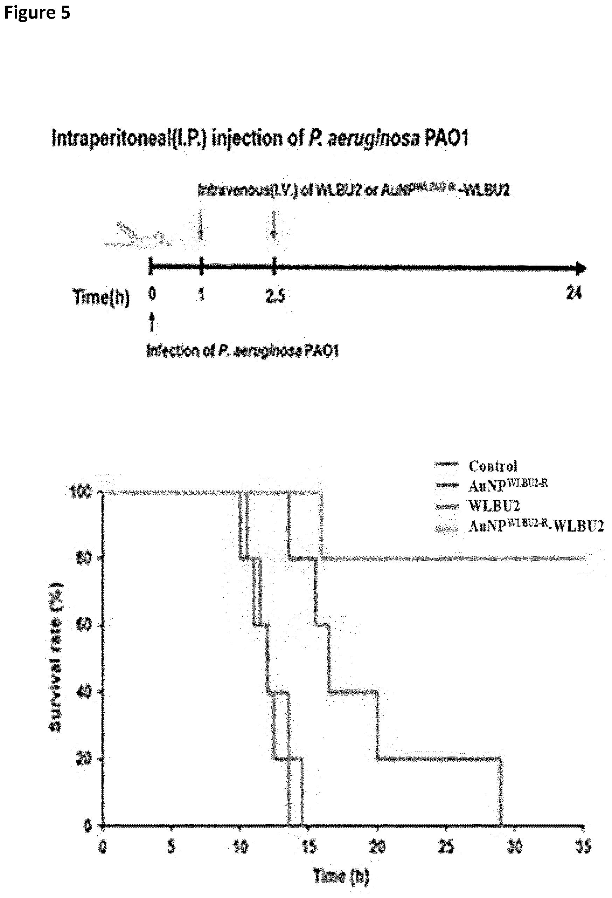

As a result, unlike the case where only the antimicrobial peptide (WLBU2) was injected, the survival rate of mice was significantly increased when the antimicrobial peptide was delivered using the delivery vehicle combining gold nanoparticles and aptamers (AuNP^{WLBU2-R}(10 nM)-WLBU2) (FIG. 5). This demonstrates that the delivery efficiency of the antimicrobial peptide is enhanced by the gold nanoparticle delivery vehicle, ultimately leading to improved antimicrobial activity.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### [Modes of the Invention]

In one aspect of the present invention, the present invention relates to an antimicrobial peptide delivery vehicle comprising a gold nanoparticle; and aptamers bound to the surface of the gold nanoparticle, wherein the aptamer comprises a base sequence of SEQ ID NO: 1 to 4 or a base sequence having 80% or more sequence identity with the sequence of SEQ ID NO: 1 to 4, and is characterized in that it specifically binds to the antimicrobial peptide WLBU2.

In the present invention, the gold nanoparticle is characterized in that it has a diameter of 10 to 20 nm.

In the present invention, the antimicrobial peptide is effective against *Pseudomonas aeruginosa, Acinetobacter baumannii, Streptococcus gordonii, Fusobacterium nucleatum, Porphyromonas gingivalis, Francisella tularensis, Yersinia pestis, Burkholderia pseudomallei, Staphylococcus aureus,* MRSA, *Enterococcus faecium, Klebsiella pneumoniae, Enterobacter cloacae, Escherichia coli,* and *Candida* species.

In one aspect of the present invention, the present invention relates to a method for preparing an antimicrobial peptide delivery vehicle, comprising the steps of developing an aptamer that specifically binds to an antimicrobial peptide WLBU2; and conjugating the aptamer to gold nanoparticles.

In the present invention, the aptamer that specifically binds to the antimicrobial peptide WLBU2 is an aptamer that includes a base sequence of SEQ ID NOS: 1 to 4 produced by SELEX or a base sequence having at least 80% sequence identity to the sequence.

In one aspect of the present invention, the present invention relates to an antimicrobial composition comprising an antimicrobial peptide delivery vehicle, comprising a gold nanoparticle; and an aptamer that specifically binds to the antimicrobial peptide WLBU2 bound to the surface of the gold nanoparticle.

In the present invention, the antimicrobial composition may be any one of a pharmaceutical, cosmetic, quasi-drug, veterinary drug, food additive, or feed additive.

## Claims

1. An antimicrobial peptide delivery vehicle comprising :
a gold nanoparticle; and
aptamers bound to the surface of the gold nanoparticle, wherein the aptamer comprises a base sequence of SEQ ID NOs: 1 to 4, or a base sequence having 80% or more sequence identity with SEQ ID NOs: 1 to 4, and specifically binds to the antimicrobial peptide WLBU2.

2. The antimicrobial peptide delivery vehicle of claim 1, wherein the gold nanoparticle has a size of 10 to 20 nm.

3. The antimicrobial peptide delivery vehicle of claim 1, wherein the antimicrobial peptide is effective against one or more microorganisms selected from the group consisting *Pseudomonas aeruginosa, Acinetobacter baumannii, Streptococcus gordonii, Fusobacterium nucleatum, Porphyromonas gingivalis, Francisella tularensis, Yersinia pestis, Burkholderia pseudomallei, Staphylococcus aureus,* MRSA, *Enterococcus faecium, Klebsiella pneumoniae, Enterobacter cloacae, Escherichia coli,* and *Candida* species.

4. A method of preparing a antimicrobial peptide delivery vehicle, comprising:
developing an aptamer that specifically binds to the antimicrobial peptide WLBU2; and
conjugating the aptamer to gold nanoparticles.

5. The method of preparing a antimicrobial peptide delivery vehicle of claim 4, wherein the aptamer that specifically binds to the antimicrobial peptide WLBU2 is an aptamer that includes a base sequence of SEQ ID NOS: 1 to 4 produced by SELEX or a base sequence having at least 80% sequence identity to the sequence.

6. An antimicrobial composition comprising:
an antimicrobial peptide delivery vehicle, which includes a gold nanoparticle and an aptamer that specifically binds to the antimicrobial peptide WLBU2, conjugated to the surface of the gold nanoparticle.

7. The antimicrobial composition of claim 6, wherein the antimicrobial composition is any one of a pharmaceutical, cosmetic, quasi-drug, veterinary drug, food additive, or feed additive.
